# EUROPEAN PATENT APPLICATION

(11) **EP 4 796 086 A1**
(43) Date of publication of application: **26.08.2026**
(21) Application number: 25823732.0
(22) Date of filing: 13.05.2025
(51) Int. Cl.: A61F 2/44

(54) **DEVICE FOR MAINTAINING INTERVERTEBRAL SPACE AND PROTECTING SPINAL CORD**

(30) Priority: 16.12.2024 KR 20240187605
(71) Applicant: Korea University Research and Business Foundation of Korea University, Seoul 02841 (KR)
(72) Inventor: AN, Sung-Jae, Seocho-gu, Seoul 06597 (KR)
(74) Representative: BCKIP Part mbB
(86) International application number: PCT/KR2025/006414
(87) International publication number: WO 2026/134456

(57) **Abstract**

The present application relates to a device for maintaining vertebral body spacing and protecting a spinal cord, the device including first and second bodies extending in a longitudinal direction, in which the first and second bodies are configured to approach each other and coupled to each other to form an internal space extending in the longitudinal direction between the first and second bodies so that the internal space accommodates a spinal cord, and in which the first and second bodies are structured to have inwardly curved ventral ends.

## Description

### [Technical Field]

The present application relates to a device(spinal corpectomy cage) for maintaining vertebral body spacing and protecting a spinal cord.

### [Background Art]

A spinal injury refers to a state in which injury occurs to various structures such as vertebral bones, discs, ligaments, muscles, and nerves. Because a spine is closely related to a nerve system, spinal injury may cause nerve injury, which may result in severe functional problems such as paralysis, pain, or sensory loss. The spinal injury may occur due to accidents, falls, sports activities, or diseases. Various symptoms, which range from mild pain to whole-body paralysis, may occur depending on the degree and types of injury. Surgical treatment varies depending on the range, degree, and types of injury and a patient's condition, and various treatment methods, such as surgery, medication, and rehabilitation, are performed in combination.

Among various treatment methods, the surgical treatment of a vertebral body includes various technical approaches for restoring fracture or abnormal deformation of the spine. Recently, various technologies are being utilized to decompress or stabilize the damaged vertebral bodies. Methods used to stabilize the vertebral body include vertebroplasty, kyphoplasty, simple decompression, posterior fixation, and corpectomy with fixation. The vertebroplasty refers to a therapeutic method that restores a fractured vertebral body and alleviates pain. In particular, the vertebroplasty is widely applied to patients with osteoporosis: Research has been actively conducted on fabrication of customized spinal implant using a 3D printing technology and on development of biomaterials that assists in physiological reconstruction of the vertebral body. In addition, these technologies assist in restoring the fractured spine and alleviating pain by means of small incisions and provide an advantage of shortening recovery time and hospitalization periods.

However, current spinal implants are imperfect in their design, material, and mechanical stability in combination with other implants when inserted in the human spine. Spinal corpectomy, which is one of the most invasive spinal surgery performed, also requires various implants including corpectomy cage, pedicle screw, crosslink, and rod. Implants utilized in the spinal corpectomy are also imperfect especially in various aspects including difficult surgical approach, necessity of including adjacent healthy segments for the disease segment, inability to protect spinal cord, and mechanical instability. Most importantly, current corpectomy cage is unstable in nature, as it has no connection with other implants, and is inserted between weak portion of upper and lower vertebral body centers with continuous application of axial loading. In the case of a polymer material such as PEEK, there is a limitation in balancing sufficient strength with fusion characteristics.

In addition, in case that a dura mater is brought into direct contact with or compressed by the device during the implantation of the device, a spinal cord may be injured, or the dura mater may be perforated, which may lead to severe complications such as postoperative neurological deficits or cerebrospinal fluid leakage.

In particular, these problems become severer in case that an implantation route and design of the device do not accurately suit the curved structure of the spinal cord or an excessive force is applied during an implantation process. Likewise, if bony fragments migrate or spinal tumor grows toward spinal cord, they can compress or irritate spinal cord and cause damage. Current spinal corpectomy cage holds this inherent risk as the cage design does not permit any spinal cord protection. Oftentimes, repeated surgical treatment is the only solution, which is difficult to implement because of the patient condition, distorted spinal anatomy from index surgery, and scar tissue formation with adhesive tissue surrounding the initially dissected surgical plane. In addition, a long-term injury may be caused even in case that the porous structure, which is designed to promote fusion of bone, comes into excessive contact with the dura mater or a sufficient interspace from the spinal cord cannot be ensured.

Furthermore, due to the large diameter of the current corpectomy cage design, spinal cord and nerve roots can be damaged while inserting the cage through narrow surgical corridor from routine posterior spinal approach. Therefore, the present application is intended to provide a device for easier and safer implant insertion by surgeon, less vertebral body needed to place the implant, better maintenance of vertebral body space and stability by achieving less implant subsidence, and permanent spinal cord protection.

The background art of the present application is disclosed in Korean Patent No. 10-2510244.

### [Disclosure]

### [Technical Problem]

The present application has been made in an effort to solve the above-mentioned problems in the related art, and an object of the present application is to provide a device for maintaining vertebral body spacing and protecting a spinal cord, the device being capable of maintaining vertebral body height and protecting the spinal cord following total or partial spinal corpectomy surgery, which involves removal of the vertebral body.

In addition, another object of the present application is to provide a device for maintaining vertebral body spacing and protecting a spinal cord, the device being capable of ensuring a long-term therapeutic effect by means of bony fusion within the patient's bony construct and with the implant and the patient's bone.

However, technical problems to be solved by the exemplary embodiment of the present application are not limited to the aforementioned technical problem, and other technical problems may be present.

### [Technical Solution]

As a technical means for achieving the technical objects, a device for maintaining vertebral body spacing and protecting a spinal cord according to an embodiment of the present application may include: first and second bodies extending in a longitudinal direction, in which the first and second bodies are configured to approach each other and coupled to each other to form an internal space extending in the longitudinal direction between the first and second bodies so that the internal space accommodates a spinal cord, and in which the first and second bodies are structured to have inwardly curved ventral ends.

In addition, according to the embodiment of the present application, inner surfaces of the first and second bodies, which are coupled to and adjoin each other, may be formed as curved surfaces.

In addition, according to the embodiment of the present application, the other end of the first body and the other end of the second body may be formed to be spaced apart from each other when the first and second bodies are coupled.

In addition, according to the embodiment of the present application, at least a part of the first body and at least a part of the second body may be formed to be symmetric based on one surface extending in the longitudinal direction.

In addition, according to the embodiment of the present application, the first and second bodies may be manufactured in ready-made sizes and selectively used to correspond to a height of the vertebral body.

In addition, according to the embodiment of the present application, dorsal surfaces of the first and second bodies may include grooves, and the grooves may be formed to accommodate a metal rod.

In addition, according to the embodiment of the present application, a cross-section of the groove may be formed in an oblique shape or a shape having a curvature.

In addition, according to the embodiment of the present application, the device for maintaining vertebral body spacing and protecting a spinal cord may further include: a support structure extending in the longitudinal direction around the vertebral body.

In addition, according to the embodiment of the present application, the support structure may include: a fixing body configured to accommodate the other end of the metal rod accommodated in the grooves of the first and second bodies; a support column extending in the longitudinal direction around the vertebral body; a fixing screw inserted into the vertebral body and configured to fix the support structure; and a coupling member configured to fix a spine by coupling the fixing screw and the support column.

In addition, according to the embodiment of the present application, the fixing body may be slidably fastened to two opposite transverse sides of the support column extending in the longitudinal direction around the vertebral body.

In addition, according to the embodiment of the present application, the fixing body may include a recessed fixing body groove having a width corresponding to a diameter of the metal rod.

In addition, according to the embodiment of the present application, the first and second bodies may include porous structures, and the porous structure may be formed on a part of the ventral surface.

The technical solution is just illustrative but should not be interpreted as being intended to limit the present application. In addition to the above-mentioned exemplary embodiment, additional exemplary embodiments may be present in the drawings and the detailed description.

### [Advantageous Effects]

According to the technical means of the present application, the present application provides the structure in which the ventral end is bluntly curved inward, such that the device for maintaining vertebral body spacing and protecting a spinal cord may be easily implanted between the vertebral bodies.

In addition, in the present application, the inner surfaces of the bodies of the device for maintaining vertebral body spacing and protecting a spinal cord, which adjoin the spinal cord, may be processed as curved surfaces, thereby reducing injury to the spinal cord and the dura mater when the device is implanted.

In addition, in the present application, the support column may be connected to the fixing body, and the two metal rods are fitted and coupled to the fixing body from above and below, thereby preventing the device for maintaining vertebral body spacing and protecting a spinal cord from moving toward the anterior side or the posterior side.

However, the effects, which can be obtained by the embodiments of the present application, are not limited to the above-mentioned effects, and other effects may be achieved.

### [Description of Drawings]

FIG. 1 is a view illustrating a spine when viewed in a lateral direction of a human body.
FIG. 2 is a view exemplarily illustrating a state in which a spinal cord is accommodated in an internal space of a device for maintaining vertebral body spacing and protecting a spinal cord according to the embodiment of the present application.
FIG. 3 is an exploded perspective view illustrating a first body and a second body according to the embodiment of the present application.
FIG. 4 is a view exemplarily illustrating a state in which the first body and the second body according to the embodiment of the present application are coupled to each other while adjoining each other.
FIG. 5 is a view exemplarily illustrating structures of ventral ends of the first and second bodies according to the embodiment of the present application.
FIG. 6 is a view exemplarily illustrating a state in which the present device is implanted into a space between vertebral bodies while spreading the space.
FIG. 7 is a view schematically illustrating a state in which a conventional cage, which is substituted for a vertebral body, is installed in an injured spine of a patient and a support structure is installed around the spine in a typical manner.
FIG. 8 is a view exemplarily illustrating a state in which metal rods are accommodated in grooves of the first and second bodies according to the embodiment of the present application.
FIG. 9 is a view exemplarily illustrating a state in which the present device, which is fastened to the support structure according to the embodiment of the present application, is implanted between vertebral bodies.
FIG. 10 is a view exemplarily illustrating the possible area of the porous structures of the first and second bodies according to the embodiment of the present application.

### [Best Mode]

Hereinafter, embodiments of the present application will be described in detail with reference to the accompanying drawings so that those with ordinary skill in the art to which the present application pertains may easily carry out the embodiments. However, the present application may be implemented in various different ways and is not limited to the embodiments described herein. A part irrelevant to the description will be omitted in the drawings in order to clearly describe the present application, and similar constituent elements will be designated by similar reference numerals throughout the specification.

Throughout the specification of the present application, when one constituent element is referred to as being "connected to" another constituent element, one constituent element can be "directly connected to" the other constituent element, and one constituent element can also be "electrically or indirectly connected to" the other element with other elements therebetween.

Throughout the specification of the present application, when one member is disposed "on", "at an upper side of", "at an upper end of", "below", "at a lower side of", or "at a lower end of" another member in the present specification of the present application, this includes not only a case where one member is brought into contact with another member, but also a case where still another member is present between the two members.

Throughout the specification of the present application, unless explicitly described to the contrary, the word "comprise" or "include" and variations, such as "comprises", "comprising", "includes" or "including", will be understood to imply the inclusion of stated constituent elements, not the exclusion of any other constituent elements.

Hereinafter, a device 1 for maintaining vertebral body spacing and protecting a spinal cord (hereinafter, referred to as 'the present device') according to the embodiment of the present application will be described. The present device, which is substituted for a part of a removed vertebral body, is partially implanted between vertebral bodies, which are spaced apart from each other in a superior-inferior direction or a proximal-distal direction, while surrounding at least a part of a spinal cord to protect the spinal cord. Therefore, anterior, posterior, medial, lateral, proximal, and distal directions may be understood by those skilled in the art based on functions of the present device. In addition, a longitudinal direction of the present device may be understood as the superior-inferior direction or the proximal-distal direction, and a transverse direction of the present device may be understood as a medial-lateral direction or a left-right direction. Hereinafter, for convenience, directions described or illustrated as being toward the medial side or lateral side may be understood by those skilled in the art as the leftward or rightward direction.

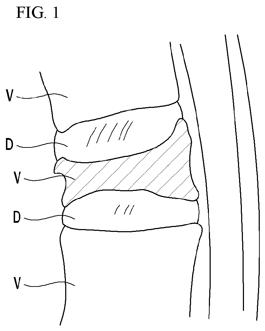

A spine includes seven cervical vertebrae, twelve thoracic vertebrae, and five lumbar vertebrae, and extends vertically along a back of a human body. A vertebral body, which is formed at the anterior side of the spine, and a posterior arch, which is formed at the posterior side, define a spinal canal at a center of the spine, and a spinal cord passes through the inside of the spinal canal. In this regard, FIG. 1 is a view illustrating a spine when viewed in a lateral direction of a human body according to an embodiment of the present application. With reference to FIG. 1, the spine may include vertebral bodies V, and intervertebral discs D disposed between the vertebral bodies. The spinal cord extending inside the spinal canal has a length of 41 to 45 cm and includes motor nerves, sensory nerves, and autonomic nerve.

A healthy vertebral body has a shape similar to a quadrangular shape. However, when spinal health deteriorates due to aging-related osteoporosis, trauma, or spinal tumors or the like, the vertebral body becomes injured and loses an original shape thereof. The vertebral body may be damaged or injured by a compression fracture or a pathologic fracture. In the case of the compression fracture caused by an excessive force applied to the vertebral body, a single vertebral body is injured typically. However, in the case of the pathologic fracture caused by cancer metastasis, one or more vertebral bodies are injured. When the vertebral body is injured, bone or tissue is pushed toward the posterior side and compresses the spinal cord inside the spinal canal. In case that the spinal cord is compressed, symptoms, such as pain or paralysis of lower limbs, may occur, which requires a surgical therapy.

According to the embodiment of the present application, the present device may support a space between vertebral bodies spaced apart from each other after a part of the vertebral body, which has a compression fracture or a pathologic fracture, is removed from a patient. Further, the present device may protect the spinal cord at the posterior side of the vertebral body so that the spinal cord is not compressed, and the present device may protect spinal nerves surrounded by a dura mater that adjoins an inner surface of the present device.

According to the embodiment of the present application, the present device may include a first and second bodies 10 and 20 extending in a longitudinal direction.

Specifically, the present device may include the first and second bodies 10 and 20 extending in the longitudinal direction, and the first and second bodies 10 and 20 may be coupled to each other and define a structure capable of maintaining an interval between vertebral bodies.

The first and second bodies 10 and 20 of the present device may approach each other and be coupled to define an internal space provided between the first and second bodies 10 and 20 and extending in the longitudinal direction so that the spinal cord is accommodated in the internal space.

Specifically, when the first and second bodies 10 and 20 of the present device approach each other and are coupled to each other, the internal space extending in the longitudinal direction may be formed between the first and second bodies 10 and 20, and the internal space may accommodate the spinal cord. When the first and second bodies 10 and 20 are coupled, the first and second bodies 10 and 20 may engage with each other and provide a stable space therein, thereby protecting the spinal cord from external pressure or injury. In addition, the coupling structure of the first and second bodies 10 and 20 may provide a fixing force between the vertebral bodies, thereby simultaneously achieving the functions of protecting the spinal cord and maintaining the stable interval.

The configuration in which the first and second bodies 10 and 20 of the present device approach each other may be understood as a configuration in which a distance between the first and second bodies 10 and 20 decreases, and at least one surface of the first body 10 and at least one surface of the second body 20 are coupled to constitute a single device. In this case, coupling methods may include fitting including loose fit, transition fit, and interference fit, and taper coupling. Further, the coupling method may further be understood as a broad concept including means for constituting a single device, such as a case in which one surface of the first body 10 and one surface of the second body 20 are joined by a biocompatible bonding agent.

Specifically, the loose fit or the intermediate fit may be used to easily adjust the device during surgery. After fixation, the device may be more securely fixed by the interference fit or the taper coupling. In addition, coupling surfaces of the first and second bodies 10 and 20 may include a groove 12a and a protruding portion 22a in order to provide an additional coupling force. In case that the biocompatible bonding agent is used, the groove 12a and the protruding portion 22a may allow the device to be accurately fixed between the vertebral bodies and provide a long-term fixing force to prevent the device from moving or changing in position after surgery. In addition, various coupling methods described above may be selectively applied in accordance with a patient's condition and surgical environments.

For example, in case that the patient has low bone density or osteoporosis, an additional fixing force may be provided by using the biocompatible bonding agent instead of the interference fit or the taper coupling. In contrast, in the case of the patient with healthy bone, the device may be safely fixed by means of the interference fit after the loose fit. Depending on the surgical environments, a simple fitting coupling method may be used to implement minimally invasive surgery, the interference fit may be used after the loose-fit when more secure fixation is required, or the device may be easily adjusted during surgery by means of a method of using both the taper coupling and the biocompatible bonding agent, and then the device is more securely fixed by means of the interference fit. A coupling surface of the device may be designed in a tapered shape to maximize a frictional force during coupling, and the taper coupling may be used together with the biocompatible bonding agent to provide a long-term fixing force.

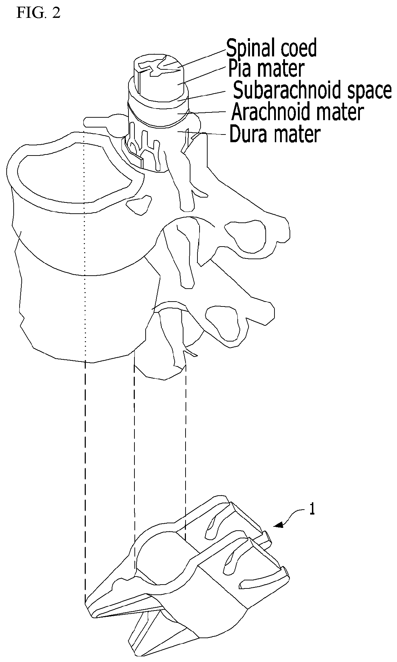

FIG. 2 is a view exemplarily illustrating a state in which a spinal cord is accommodated in an internal space of the device 1 for maintaining vertebral body spacing and protecting a spinal cord (the present device) according to the embodiment of the present application.

With reference to FIG. 2, at least a part of the first body 10 and at least a part of the second body 20 may be formed to cover or surround a part of the spinal canal in the patient's spine. Because the spinal cord is positioned in the internal space (cylindrical space) formed as the first and second bodies 10 and 20 adjoin each other, the spinal cord may be injured when the present device is implanted between the vertebral bodies, and a sharp edge of an inner surface or the protruding portion 22a of the present device may cause direct pressure or friction burn to the spinal cord. In particular, when the sharp edge compresses the spinal cord while coming into contact with the spinal cord, a portion of tissue may be pressed or scraped, which may cause bleeding, inflammation, or, in severe cases, nerve injury.

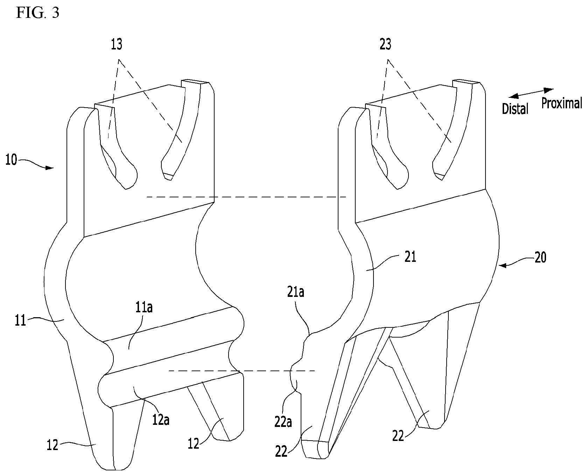

FIG. 3 is an exploded perspective view illustrating the first and second bodies 10 and 20 according to the embodiment of the present application.

With reference to FIG. 3, the first body 10 may include a first sleeve 11, first support portions 12, and first grooves 13. The first sleeve 11 may extend in the longitudinal direction and define the internal space by being coupled to a second sleeve 21, as described below. The first sleeve 11 may extend in the longitudinal direction while having a curved shape in order to cover a predetermined portion of the spinal canal in the patient's spine. The first sleeve 11 may have a convex outer peripheral surface formed outside the sleeve, a concave inner peripheral surface formed inside the sleeve, a first curved surface 11a configured to adjoin the second sleeve 21, and a recessed surface 12a coupled to the second sleeve 21. The internal space formed by coupling the first sleeve 11 and the second sleeve 21 may have a hollow shape extending in a cylindrical shape, and the internal space may have a radius of about 10 mm. As described below, the first and second sleeves 11 and 21 may be provided to have different sizes so that the radius of the internal space varies.

In addition, with reference to FIG. 3, the first sleeve 11 of the present device may have the recessed surface 12a configured to correspond to the protruding portion 22a of the second sleeve 21 to be described below. The recessed surface 12a may be formed complementarily with the protruding portion 22a of the second sleeve 21. A surgeon may couple the recessed surface 12a of the first sleeve 11 and the protruding portion 22a of the second sleeve 21 and couple the first sleeve 11 and the second sleeve 21 based on the coupling surfaces. The recessed surface 12a may be understood as having an appropriate shape that mediates the coupling with the protruding portion 22a.

The second sleeve 21 of the present device may be substantially identical to the first sleeve 11 and formed symmetrically with the first sleeve 11. As illustrated in FIG. 3, the protruding portion 22a, which corresponds to the recessed surface 12a, may be formed on the coupling surface.

The protruding portion 22a may be provided on one surface of the second sleeve 21. In the embodiment, the protruding portion 22a may extend in the longitudinal direction along the coupling surface of the second sleeve 21 coupled to the first sleeve 11. A cross-sectional shape of the protruding portion 22a protruding from the coupling surface of the second sleeve 21 may be one of a circular shape having a predetermined central angle, a semi-circular shape, a convex quadrangular shape, and a shape tapered toward an end thereof, or may be another cross-sectional shape formed to facilitate the coupling with the first sleeve 11.

With reference to FIG. 3, the first sleeve 11 may have the convex first curved surface 11a provided at an upper end of the recessed surface 12a and formed in the longitudinal direction along the concave inner peripheral surface formed inside the first sleeve 11, and the second sleeve 21 may have a convex second curved surface 21a provided at an upper end of the protruding portion 22a and formed in the longitudinal direction along a concave inner peripheral surface formed inside the second sleeve 21.

The first support portions 12 extend in one direction from two opposite longitudinal sides of the first sleeve 11. The first support portions 12 may extend from two opposite longitudinal sides of the first sleeve 11, which surround a part of the spinal canal, toward the anterior side at which a vertebral body, from which at least a part thereof is removed, is positioned, such that surfaces of the first support portions 12, which are formed at two opposite longitudinal ends of the first support portions 12 between the adjacent vertebral bodies, may come into contact with the vertebral bodies. Therefore, the first support portions 12 may be implanted between the vertebral bodies V, which are spaced apart from each other at the two opposite longitudinal sides of the first sleeve 11, and the first support portions 12 may support the vertebral bodies. A length by which the first support portions 12 extend from the two opposite longitudinal ends of the first sleeve 11 toward the anterior side may be about 20 mm, and the extension length may vary depending on shapes and sizes of spines that vary depending on patients.

With reference to FIG. 3, the first support portion 12 may be formed such that the extension lengths and widths of the surfaces of the two opposite longitudinal ends, which adjoin the vertebral body, are largest, and an extension length L and a width W, which corresponds to the extension length, are gradually decreased toward the anterior side of the support portion. That is, an area of a cross-section of the first support portion 12, which is perpendicular to the longitudinal direction, may be gradually decreased longitudinally inward from the two opposite longitudinal ends of the first sleeve 11. The first support portion 12 may have a softly curved shape along a periphery thereof in order to prevent stress, which is applied to the first support portion 12 when the first support portion 12 supports the vertebral body V, from being concentrated on one point on the first support portion 12. The area of the cross-section of the first support portion 12 is gradually decreased longitudinally inward, such that a load or stress transmitted from the adjacent vertebral body may be transmitted to a support column 32 to be described below.

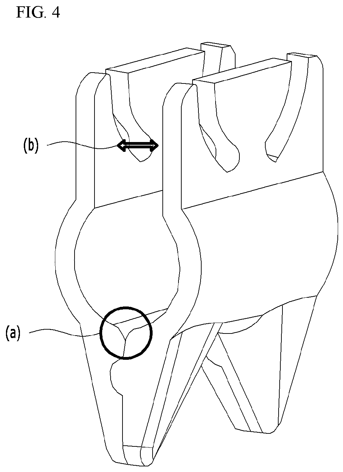

FIG. 4 is a view exemplarily illustrating a state in which the first and second bodies 10 and 20 according to the embodiment of the present application are coupled to each other while adjoining each other.

With reference to FIG. 4A, inner surfaces of the first and second bodies 10 and 20 of the present device, which extend in the longitudinal direction, are formed as curved surfaces and coupled to define the space that may accommodate therein the spinal cord. In other words, similar to the configuration in which the first sleeve 11 is coupled to the second sleeve 21, the first support portions 12 may be coupled to second support portions 22. The adjoining inner surface may be formed as a curved surface and protect the spinal cord or the dura mater, which surrounds the spinal cord, when the present device is implanted.

Specifically, the first curved surface 11a of the first body 10 and the second curved surface 21a of the second body 20 may adjoin each other, and the inner surfaces may be formed as curved surfaces, thereby minimizing friction with the spinal cord and reducing local pressure applied to the spinal cord.

With reference to FIG. 4B, the present device may be formed such that the other end of the first body 10 and the other end of the second body 20 are spaced apart from each other when the first and second bodies 10 and 20 are coupled.

Specifically, as rear ends of the first and second bodies 10 and 20 are spaced apart from each other, the present device may have an opening extending in the longitudinal direction from rear sides of the first and second bodies 10 and 20.

At least a part of the first body 10 and at least a part of the second body 20 of the present device may be formed to be symmetric with respect to one surface extending in the longitudinal direction.

Specifically, the first and second bodies 10 and 20 may constitute a symmetric structure, thereby providing uniform pressure and improving stability between the vertebral bodies when the first and second bodies 10 and 20 are coupled. The symmetric configuration of the first and second bodies 10 and 20 may allow the device to be accurately positioned after implantation without being biased, and the symmetric configuration may prevent unnecessary injury by uniformly dispersing pressure applied to the spinal cord. The symmetric structure may provide the same fixing force in the left-right direction, such that the device may be stably maintained without being swayed by a fine motion or external impact between the vertebral bodies.

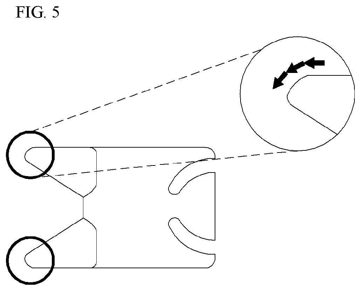

FIG. 5 is a view exemplarily illustrating structures of ventral ends of the first and second bodies 10 and 20 according to the embodiment of the present application.

With reference to FIG. 5, the ventral ends of the first and second bodies 10 and 20 of the present device may have structures curved inward.

Specifically, the inwardly curved portions of the ventral ends of the first and second bodies 10 and 20 may each be smoothly connected by a curved line and each have a shape in which the thickness of an end is gradually decreased. A curvature of the curved portion may be optimized so that the device may naturally engage with the vertebral bodies when the device is implanted between the vertebral bodies. For example, the curved line of the end may be designed to be consistent with a curvature of the vertebral body.

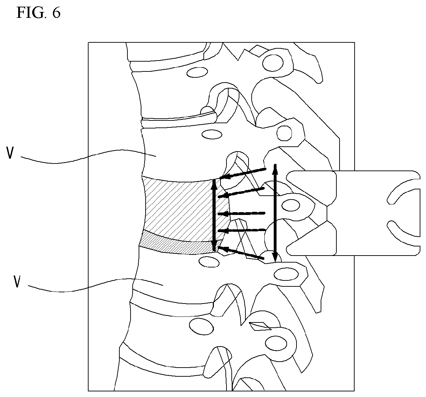

FIG. 6 is a view exemplarily illustrating a state in which the present device is implanted into a space between vertebral bodies while spreading the space.

With reference to FIG. 6, even though a height of the device implanted from the dorsal side is slightly higher than a height of a resected portion of the vertebral body, the ventral ends of the first and second bodies 10 and 20 have the inwardly curved structures, such that the device may be naturally implanted while spreading the space between the vertebral bodies.

According to the embodiment of the present application, the first and second bodies 10 and 20 may be manufactured in ready-made sizes and selectively used while corresponding to a height of the vertebral body.

Specifically, the first and second bodies 10 and 20 may be pre-manufactured in various sizes (ready-made sizes) and designed such that a suitable size may be selected depending on the intervals between the patient's vertebral bodies. A surgeon may apply the device in a customized manner in consideration of individual anatomical structures and conditions of the patients during surgery.

For example, the present devices may be provided in various forms having different heights and curvatures in accordance with the heights of the vertebral bodies, thereby improving convenience for the surgeon. Further, the device may accurately engage within the patient's body, thereby providing a stable structure.

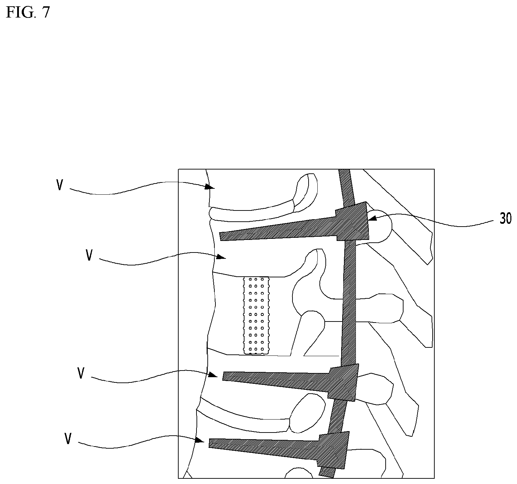

FIG. 7 is a view schematically illustrating a state in which a conventional cage, which is substituted for a vertebral body, is installed in an injured spine of a patient and a support structure 30 is installed around the spine in a typical manner.

With reference to FIG. 7, a part of the vertebral body may be removed from the posterior side of the patient, a cage, which is made of a biocompatible material such as titanium and substituted for the removed vertebral body, may be implanted between the adjacent vertebral bodies V, and then the support structure 30 may be provided around the spine to ensure stability.

Specifically, the cage is implanted between the adjacent vertebral bodies V while replacing the removed vertebral body and serves to maintain the space between the vertebral bodies and stabilize the axis of the spine. The cage may be made of titanium or the like with biocompatibility so that the long-term stability thereof may be maintained in the human body. The cage may be designed as a structure capable of providing sufficient strength to withstand pressure between the vertebral bodies and enhancing the fixing force by means of an interaction with surrounding tissue. In addition, the cage may be designed to have the same height as the removed vertebral body to accurately adjust the spacing between the vertebral bodies. In order to improve the stability of the device after implantation, the surface of the cage, which comes into contact with the vertebral body, may be subjected to surface treatment in consideration of bond ability with biological tissue.

After the implantation of the cage, the support structure 30 which typically refers to the spinal screws and rods system to be described below may be provided. The support structure 30 may extend in the longitudinal direction along two opposite sides of the vertebral body and provide an additional fixing force while assisting in coupling the vertebral body and the cage. The support structure 30 is positioned at a posterior side of the vertebral body and serves to prevent the vertebral body from moving. In addition, the support structure 30 may be positioned at a lateral side of the vertebral body and reinforce the coupling between the cage and the vertebral body.

According to the embodiment of the present application, dorsal surfaces of the first and second bodies 10 and 20 include grooves, and the grooves may be formed to accommodate a metal rod 40.

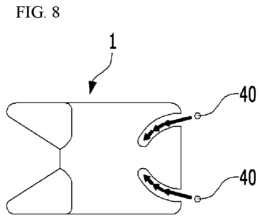

FIG. 8 is a view exemplarily illustrating a state in which the metal rods 40 are accommodated in the grooves of the first and second bodies 10 and 20 according to the embodiment of the present application.

With reference to FIG. 8, the grooves each having an oblique shape extending in the longitudinal direction or a shape having a curvature may be formed in the dorsal surfaces of the first and second bodies 10 and 20. The groove has a structure designed to accommodate the metal rod 40. A cross-section of the groove has a curvature, and the groove is formed such that the metal rod 40 is stably positioned.

The cross-section of the groove has an oblique shape or a shape having a curvature and provides an insertion space in the dorsal surface. A depth and width of the groove may be designed to accurately suit a diameter of the metal rod 40. The grooves of the first and second bodies 10 and 20 may have symmetric structures. The metal rods 40 serve to connect the first and second bodies 10 and 20 in the grooves. The cross-sections of the grooves may be consistent with the diameter of the metal rod 40 and formed to be continuously connected while maintaining predetermined intervals from the dorsal surfaces.

The present device may include the support structure 30 extending in the longitudinal direction around the vertebral body.

The support structure 30 of the present device may include a fixing body 31 configured to accommodate the other end of the metal rod 40 accommodated in the grooves of the first and second bodies 10 and 20, the support column 32 extending in the longitudinal direction around the vertebral body, a fixing screw 33 configured to be inserted into the vertebral body and fix the support structure 30, and a coupling member 34 (screw head)configured to couple the fixing screw 33 and the support column 32 to fix the spine.

The fixing body 31 accommodates the other end of the metal rod 40 in the grooves of the first and second bodies 10 and 20. The fixing body 31 is connected to the support column 32 and stably fixes the present device. The fixing body 31 may include a groove or a hole corresponding to the diameter of the metal rod 40. The fixing body 31 may be formed to be tightly attached to the metal rod 40 so that the metal rod 40 does not slip inside the fixing body 31. In addition, a portion of the fixing body 31, which is coupled to the support column 32, may be processed in a quadrangular shape, such that the fixing body 31 may be easily assembled with the support structure 30.

The fixing body 31 of the present device may include a recessed fixing body groove (or hole) having a width corresponding to the diameter of the metal rod 40.

Specifically, the fixing body 31 may have a main body structure that may have various shapes. The fixing body 31 may have the fixing body groove (or hole) curvedly recessed to accommodate the metal rod 40, and an internal space into which the support column 32 is inserted, the internal space having a cylindrical shape or a partially cylindrical shape. The fixing body groove (or hole) may have a width and depth corresponding to the diameter of the metal rod 40. The fixing body groove (or hole) may be designed so that the metal rod 40 is accurately positioned in the fixing body groove (or hole). The internal space having a cylindrical shape or a partially cylindrical shape is processed to correspond to a diameter of the support column 32, such that the support column 32 may be stably fixed without swaying after the support column 32 is inserted into the internal space. A cross-section of the cylindrical space may have a circular shape, and an integrated structure may be implemented as the support column 32 is coupled to the fixing body 31. The interior of the fixing body 31 may be processed in a flat shape, a curved shape, or a screw thread shape into which a screw may be inserted and tightened in consideration of fastening to the support structure 30 and to the groove (or hole) accommodating the metal rod 40.

In addition, the fixing bodies 31 may be slidably fastened to two opposite transverse sides of the support column 32 extending in the longitudinal direction around the vertebral body.

Specifically, the fixing bodies 31 may be positioned over the two opposite transverse sides of the support column 32 extending in the longitudinal direction around the vertebral body and fastened so as to be slidable on the support column 32. A fastening portion configured to be coupled to the support column 32 may be formed at a lower end of the fixing body 31, and the fastening portion may be tightly attached to a transverse section of the support column 32. After the fixing body 31 is moved in a transverse direction on the support column 32, the fixing body 31 may be fixed to the support column 32 by using screw.

The fixing body 31 may be formed such that an internal space of the fastening portion is consistent with a cross-section of the support column 32 so that the fixing body 31 may be movable along a length of the support column 32. A position of the fixing body 31 may be finely adjusted as the fixing body 31 is slid.

The support column 32 has a structure extending in the longitudinal direction around the vertebral body and coupled to the fixing body 31. The support column 32 has a cylindrical shape. An outer surface of the support column 32 may be processed to be smooth. Alternatively, in order to enhance the fastening force as necessary, fine protrusions or grooves may be added to the outer surface of the support column 32. A length of the support column 32 may be adjusted in accordance with a height of the vertebral body.

In addition, the two support columns 32 may extend in the proximal-distal direction from two opposite outer sides or left/right sides based on the patient's spine. A distance between the support columns 32 may vary depending on the patient's body type or the spinal environment.

The fixing screw 33 is inserted into the vertebral body and securely fixes the support column 32 and the coupling structure. A body of the screw may have a spiral screw thread formed in the longitudinal direction. The screw thread may be formed to correspond to bone density and strength of the vertebral body. The other end of the fixing screw 33 may be manufactured in a pointy shape, such that the fixing screw 33 may easily penetrate the vertebral body during insertion. A head of the fixing screw 33 may have a hexagonal or cross-shaped structure, such that the fixing screw 33 may be easily coupled to an insertion tool, and the fixing screw 33 may come into flat contact with and be fastened to the support column 32 or the coupling member 34. In addition, the fixing screw 33 may be made of titanium alloy or stainless steel to ensure strength and durability, and surface treatment (e.g., an oxidation film) may be added in consideration of usage inside the human body.

The coupling member 34 is a structure that serves to integrate the vertebral body and the support structure 30 by connecting the fixing screw 33 and the support column 32. The coupling member 34 is designed in a " 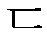 " shape or an "L" shape and fastens an upper end of the support column 32 and the fixing screw 33. A fastening groove, into which the fixing screw 33 may be inserted, may be formed at a center of the coupling member 34 and have a circular or elliptical shape.

In summary, the support structure 30 may fix the spine by inserting the fixing screw 33 into the vertebral body, extending the support column 32 in the superior-inferior direction or the proximal-distal direction, and then coupling the fixing screw 33 and the support column 32 by means of the coupling member 34 or the like.

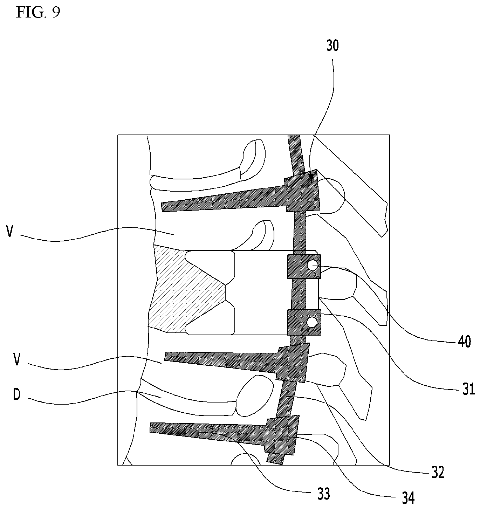

FIG. 9 is a view exemplarily illustrating a state in which the present device, which is fastened to the support structure 30 according to the embodiment of the present application, is implanted between vertebral bodies.

With reference to FIG. 9, the present device is implanted between the vertebral bodies V to maintain the spacing between the vertebral bodies V and fastened to the support structure 30 to improve the stability of the vertebral body. The present device is disposed tightly between the vertebral bodies. The fixing bodies 31 of the support structure 30 are connected by the metal rod
40 coupled to the groove positioned in the ventral surface of the present device. The support structure 30 includes the fixing screw 33 and the support column 32 extending in the longitudinal direction. The support column 32 is connected to the fixing bodies 31 at the two opposite sides of the vertebral bodies V, and the fixing screws 33 fix the lower end of the support column 32 to a vertebral body D and stably maintain the structure. The coupling member 34 securely connects the fixing screw 33 and the support column 32 and further enhances the coupling between the support structure 30 and the vertebral body.

The first and second bodies 10 and 20 of the present device may include porous structures, and the porous structure may be formed on at least a part of an outer surface of the present device. In other words, the porous structure may be formed on a portion of a surface of the present device that comes into contact with the patient's bone.

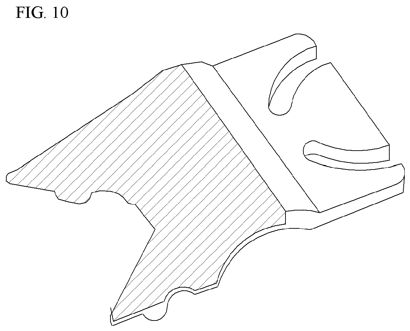

In this regard, FIG. 10 is a view exemplary illustrating the possible area of the porous structures of the first and second bodies 10 and 20 according to the embodiment of the present application.

With reference to FIG. 10, portions of the ventral surfaces of the first and second bodies 10 and 20 form the porous structures having irregular patterns, and the porous structures are distributed over the entire surfaces. The porous structure may include fine holes and bends, thereby increasing a surface area and enhancing a physical coupling force. In other words, the porous structure has a complex, irregular pattern and includes micro holes and bends on the surfaces to increase adhesion between the device and the vertebral bodies. The porous structure is formed on the ventral surfaces in consideration of direct coupling with the vertebral bodies and may provide a structural basis that may be coupled to bones, bone graft materials, or biocompatible bonding materials inside the human body. In addition, the porous structure may also be coupled to bone cement used in an environment in the human body, and the cement may permeate into the porous structure to form strong mechanical coupling between the device and the vertebral bodies.

It will be appreciated that the embodiments of the present application have been described above for purposes of illustration, and those skilled in the art may understand that the present application may be easily modified in other specific forms without changing the technical spirit or the essential features of the present application. Therefore, it should be understood that the above-described embodiments are illustrative in all aspects and do not limit the present application. For example, each component described as a single type may be carried out in a distributed manner. Likewise, components described as a distributed type can be carried out in a combined type.

The scope of the present application is represented by the claims to be described below rather than the detailed description, and it should be interpreted that the meaning and scope of the claims and all the changes or modified forms derived from the equivalent concepts thereto fall within the scope of the present application.

## Claims

1. A device for maintaining vertebral body spacing and protecting a spinal cord, the device comprising:
first and second bodies extending in a longitudinal direction,
wherein the first and second bodies are configured to approach each other and coupled to each other to form an internal space extending in the longitudinal direction between the first and second bodies so that the internal space accommodates a spinal cord, and
wherein the first and second bodies are structured to have inwardly curved ventral ends.

2. The device of claim 1, wherein inner surfaces of the first and second bodies, which are coupled to and adjoin each other, are formed as curved surfaces.

3. The device of claim 2, wherein the other end of the first body and the other end of the second body are formed to be spaced apart from each other when the first and second bodies are coupled.

4. The device of claim 3, wherein at least a part of the first body and at least a part of the second body are formed to be symmetric based on one surface extending in the longitudinal direction.

5. The device of claim 1, wherein the first and second bodies are manufactured in ready-made sizes and selectively used to correspond to a height of the vertebral body.

6. The device of claim 1, wherein dorsal surfaces of the first and second bodies comprise grooves, and the grooves are formed to accommodate a metal rod.

7. The device of claim 6, wherein a cross-section of the groove is formed in an oblique shape or a shape having a curvature.

8. The device of claim 7, further comprising:
a support structure extending in the longitudinal direction around the vertebral body.

9. The device of claim 8, wherein the support structure comprises:
a fixing body configured to accommodate the other end of the metal rod accommodated in the grooves of the first and second bodies;
a support column extending in the longitudinal direction around the vertebral body;
a fixing screw inserted into the vertebral body and configured to fix the support structure; and
a coupling member configured to fix a spine by coupling the fixing screw and the support column.

10. The device of claim 9, wherein the fixing body is slidably fastened to two opposite transverse sides of the support column extending in the longitudinal direction around the vertebral body.

11. The device of claim 10, wherein the fixing body comprises a recessed fixing body groove having a width corresponding to a diameter of the metal rod.

12. The device of claim 1, wherein the first and second bodies comprise porous structures, and the porous structure is formed on at least a part of an outer surface of the device.
